# EUROPEAN PATENT SPECIFICATION

(11) **EP 0 618 285 B1**
(45) Date of publication and mention of the grant of the patent: **16.12.1998**
(21) Application number: 94103590.9
(22) Date of filing: 09.03.1994
(51) Int. Cl.: C11B 9/02, A61K 7/46

(54) **Treated labdanum oil, process for producing the same and perfume composition containing the game**
Behandeltes Labdanumöl, Verfahren zur Herstellung desselben und dieses enthaltende Parfumzusammensetzung
Huile de labdanum traitée, procédé pour ce faire et composition de parfum la comprenant

(30) Priority: 01.04.1993 JP 75660/93
(43) Date of publication of application: 05.10.1994
(73) Proprietor: Kao Corporation, Chuo-Ku Tokyo 103 (JP)
(72) Inventor: Yamamoto, Junko, Wakayama-shi, Wakayama (JP); Tanaka, Shigeyoshi, Nagoya-shi, Aichi (JP); Koshino, Junji, Naga-Gun, Wakayama (JP); Tajima, Katsuhiko, Mihama-ku, Chiba-shi, Chiba (JP); Toi, Nao, Sakura-shi, Chiba (JP); Fujikura,Yoshiaki, Utsunomiya-shi, Tochigi (JP)
(74) Representative: Hansen, Bernd, Dr. Dipl.-Chem.

(56) References cited:
- EP-A- 0 481 521
- DE-C- 122 097
- FR-A- 679 774
- US-A- 4 464 290
- S.ARCTANDER 'Perfume and Flavor Materials of Natural Origin' 1960 , S.ARCTANDER , ELIZABETH,N.J. pages326-335 * the whole document *
- K.BAUER ET AL. 'Common fragrance and flavor materials' 1985 , VCH , WEINHEIM page 156 * page 156, line 11 *

## Description

### FIELD OF THE INVENTION

This invention relates to a treated labdanum oil with an improved odor which is useful as a material of a perfume composition, a process for producing the same, and a perfume composition containing the same.

### BACKGROUND OF THE INVENTION

Labdanum raw material is a black and highly viscous liquid obtained from plants belonging to the genus Cistus and is used in various perfume compositions. Labdanum raw material most in use includes labdanum gum obtained by collecting a resin separated from Cistus ladaniferus by boiling it in water, labdanum extract obtained by removing inorganic solids from the above-mentioned labdanum gum with an organic solvent, and labdanum gum distillate obtained by distillation or fractional distillation of the above-mentioned labdanum extract.

However, these conventional labdanum products not only give off an undesired wood-like crude odor like cedarwood oil as well as an amber-like fragrance but assume a black to brown black color. Therefore, when applied as a material of perfume compositions for soaps, detergents, etc., they deteriorate the odor and color of the scented products. It has thus been demanded to develop a treated labdanum oil having an improved odor while retaining the amber-like odor inherent to labdanum and is useful as a material of perfume compositions.

### SUMMARY OF THE INVENTION

As a result of extensive study, the present inventors have found that the offensive odor emitted from labdanum gum, etc. comes from acids, such as carboxylic acids and phenols, and that these acids can be removed by contact with an alkaline substance. The present invention has been completed based on this finding.

Namely, the present invention provides a treated labdanum oil having an acid value of not more than 10.

The present invention also provides a process for producing a treated labdanum oil having an acid value of not more than 10, which comprises contacting at least one labdanum raw material selected from:
(a) labdanum gum obtainable by collecting a resin separated from Cistus ladaniferus by boiling it in water,
(b) labdanum extract obtainable by extracting the labdanum gum with an organic solvent to remove inorganic solids, and
(c) labdanum gum distillate obtainable by distillation or fractional distillation of the labdanum gum extract,
with an alkaline substance in an amount of 0.5 to 10 equivalents, based on the acid equivalent of the labdanum raw material, and then recovering the treated labdanum oil.

The present invention further provides a perfume composition containing a treated labdanum oil having an acid value of not more than 10.

### DETAILED DESCRIPTION OF THE INVENTION

The treated labdanum oil having an acid value of not more than 10 according to the present invention can be obtained by the following process.

Labdanum gum (a) which can be used as a labdanum raw material is a resin which is separated on boiling of Cistus ladaniferus in water. Labdanum gum (a) can be obtained in a conventional manner as disclosed, for example, in the pamphlet of BIOLANDES and S. Arctander, Perfume and Flavor Materials of Natural Origin, published by Elizabeth (1960). More specifically, labdanum gum (a) can be obtained by heating the plant in water and collecting the floating oily content. In this instance, an aqueous alkaline solution may be used in place of water and the resulting solution may be neutralized with sulfuric acid, followed by collecting the floating oily content. Commercially available labdanum gum may be used as such. Labdanum extract (b) which can be used as a labdanum raw material is obtainable by dissolving labdanum gum (a) in an organic solvent, such as ethanol, and removing the insoluble inorganic solid component. A commercially available labdanum extract with organic solvent may be used as such. Labdanum gum distillate (c) which can be used as a labdanum raw material is obtained by subjecting labdanum extract (b) to distillation or fractional distillation under conditions of, for example, 140 to 240°C in column inner temperature under the vacuum degree of 2 Torr. Labdanum raw materials (a), (b) and (c) usually have an acid value of from about 18 to 120. For example, Kaori no Hyakka, published by Asakura Shoten (1989), discloses that the value is from 18 to 86; while the present inventors found that the value of labdanum gum is 111. As stated above, these raw materials have a cedarwood-like woody offensive odor.

According to the process of the present invention, one or more of labdanum raw materials (a), (b) and (c) is/are brought into contact with an alkaline substance, and preferably an aqueous alkali solution, followed by mixing with stirring.

The alkaline substance which can be used in the present invention is not particularly limited as long as it is capable of neutralizing the above-mentioned acids, such as carboxylic acids and phenols, to form water-soluble neutralized salts. In particular, one or two or more of alkali metal hydroxides, alkaline earth metal hydroxides, and alkali metal carbonates is/are preferred, with sodium hydroxide and potassium hydroxide being more preferred.

The alkaline substance is used in an amount of from 0.5 to 10 times equivalents, preferably from 1.0 to 5.0 times equivalents, based on the acid equivalent of the labdanum raw material. The acid equivalent of the labdanum raw material is calculated based on the acid value of the labdanum raw material.

While not limiting, the mixing with stirring is preferably carried out at a temperature of from 0 to 150°C, preferably from 50 to 100°C, for a period of from 0.1 to 5 hours, preferably from 0.5 to 2 hours.

The treatment with the alkaline substance may preferably be carried out in the presence of an organic solvent. The organic solvent may be added to the labdanum raw material in advance, or to the mixture of the labdanum raw material with the alkaline substance. The organic solvent to be used is not particularly limited as long as it is stable under an alkaline condition. Examples of the suitable organic solvent include hexane, heptane, cyclohexane, benzene, toluene, xylene, and mixtures thereof.

The resulting reaction mixture is then separated into an aqueous phase and an oily phase in a conventional manner, and the aqueous phase is removed. If desired, the oily phase is washed with water and distilled in a conventional manner.

The thus obtained treated labdanum oil has an acid value of not more than 10, has a markedly improved odor, and is substantially colorless. It is preferable to further subject the treated labdanum oil to precise fractional distillation to obtain a less colored and less viscous product.

The perfume composition according to the present invention comprises the above-mentioned treated labdanum oil. If desired, the perfume composition may contain other components, such as diethyl phthalate, dipropylene glycol, ethylene diglycol, Hercolyn D (methyl dihydroabietate), and natural perfumes, such as sandalwood oil and patchouli oil.

The ratio of the treated labdanum oil in the perfume composition of the present invention is preferably in the range of from 0.5 to 10 % by weight.

The perfume composition of the present invention can be prepared by mixing the treated labdanum oil and other optional components with stirring.

According to the present invention, a treated labdanum oil with a pure amber-like fragrance and a greatly reduced color can be obtained through simple operation and at low cost. The perfume composition containing the treated labdanum oil is suited for use in soaps, detergents, and the like.

The present invention will now be illustrated in greater detail with reference to Examples, but the present invention should not be construed as being limited thereto. All the parts and percents are by weight unless otherwise indicated.

### EXAMPLE 1

In a 500 cc four-necked flask equipped with a stirrer, a thermometer, and a Dimroth condenser were charged 200 g of a commercially available labdanum extract "Labdanum Resinoid" (a product of BIO Co.; acid value: 58; acid equivalent: 0.21), 200 g of a 10 % aqueous solution of sodium hydroxide (0.50 equivalent), and 400 g of toluene, and the mixture was stirred at 90°C for 30 minutes in nitrogen atmosphere. The mixture was allowed to stand for phase separation. The aqueous layer was removed and 400 g of toluene was added thereto. The resulting mixture was stirred in the same manner as described above and allowed to stand for phase separation. After removing the aqueous layer, the organic layer was combined with the previously obtained organic layer and a 10% aqueous solution of mirabilite was added to the combined organic layer. The aqueous layer thus formed was adjusted to pH of 6 to 8 with 2 % sulfuric acid, and allowed to stand. After removing the aqueous layer, toluene was removed from the organic layer by distillation to obtain 80 g of a crude treated labdanum oil having an acid value of 0.92. The resulting crude treated labdanum oil had a clean amber-like fragrance without being accompanied by a cedarwood-like unpolished odor as compared with the starting labdanum extract.

The crude treated labdanum oil was subjected to Smith distillation (140°C → 185°C/0.05 Torr) to recover 50 g of a treated labdanum oil having an acid value of 0.17. The product showed further improvements in color and viscosity.

### EXAMPLE 2

In a 300 cc four-necked flask equipped with a stirrer, a thermometer, and a Dimroth condenser were charged 50 g of a distillate in Smith distillation (185°C/0.05 Torr) of a commercially available labdanum extract "Labdanum Resinoid" (a product of CMA Co.; acid value: 80; acid equivalent: 0.071), 50 g of a 10 % aqueous solution of sodium hydroxide (0.125 equivalent), and 100 g of toluene, and the mixture was stirred at 60°C for 30 minutes in a nitrogen atmosphere. The mixture was allowed to stand for phase separation, and the aqueous layer was removed. To the organic layer was added 50 g of a 10 % aqueous solution of sodium hydroxide, and the mixture was stirred in the same manner as described above and allowed to stand for phase separation. After removing off the aqueous layer, the resulting organic layer was treated with a 10 % aqueous solution of mirabilite and toluene was removed therefrom in the same manner as in Example 1, whereby 7 g of a treated labdanum oil having an acid value of 3.1 was obtained as a distillate. The resulting treated labdanum oil had a cleaner amber-like fragrance as compared with the starting labdanum raw material.

### COMPARATIVE EXAMPLE 1

A treated labdanum oil was prepared in the same manner as in Example 2, except for using 50 g of a 1 % sodium hydroxide aqueous solution (0.0125 equivalent) as a neutralizing agent. The yield was 14 g. The resulting treated labdanum oil had an acid value of 45 and still gave out a cedarwood-like odor as compared with the product obtained in Example 2.

### EXAMPLE 3 AND COMPARATIVE EXAMPLE 2

A perfume composition was prepared from the components shown in Table 1 below in a usual manner.

**TABLE 1**

| Composition (part) | Example 3 | Comparative Example 2 |
|---|---|---|
| Vanillin | 400 | 400 |
| Patchouli oil | 20 | 20 |
| Sandalwood Mysore | 30 | 30 |
| Benzoin | 40 | 40 |
| Cinnamic acid | 20 | 20 |
| Pearlide* | 10 | 10 |
| Acetanilide | 380 | 380 |
| Labdanum extract (Labdanum Resinoid, a product of CMA Co.) | - | 100 |
| Treated labdanum oil of Example 1 | 100 | - |

| | | |
|---|---|---|
| Note: *: 1,3,4,6,7,8-Hexahydro-4,6,6,7,8,8-hexamethyl-cyclopenta-2-benzopyran, produced by Kao Corporation. | | |

The composition of Example 3 had a pure ambergrislike fragrance with little rough woody offensive odor as compared with the comparative composition. When the composition of Example 3 was incorporated into soap, the degree of coloration of the soap was remarkably reduced.

## Claims

1. A treated labdanum oil having an acid value of not more than 10.

2. A process for producing a treated labdanum oil having an acid value of not more than 10 comprising a step of contacting at least one labdanum raw material selected from:
(a) labdanum gum obtainable by collecting a resin separated from Cistus ladaniferus by boiling it in water,
(b) labdanum extract obtainable by extracting said labdanum gum with an organic solvent to remove inorganic solids, and
(c) labdanum gum distillate obtainable by distillation or fractional distillation of said labdanum gum extract,
with an alkaline substance in an amount of 0.5 to 10 equivalents, based on the acid equivalent of said labdanum raw material.

3. The process of claim 2, wherein said alkaline substance is selected form alkali metal hydroxides, alkaline earth metal hydroxides, alkali metal carbonates, and a mixture of them.

4. The process of claim 3, wherein said alkaline substance is sodium hydroxide or potassium hydroxide.

5. The process of any of claims 2, 3 and 4, wherein said labdanum raw material is contacted with said alkaline substance by adding an aqueous solution of said alkaline substance to said labdanum raw material, and then mixing with stirring the mixture.

6. The process of claim 2, wherein said labdanum raw material is contacted with said alkaline substance in the presence of an organic solvent.

7. The process of claim 6, wherein said organic solvent is selected from hexane, heptane, cyclohexane, benzene, toluene, xylene and a mixture of them.

8. A perfume composition containing a treated labdanum oil having an acid value of not more than 10.

## Patentansprüche

1. Behandeltes Labdanumöl mit einem Säurewert von nicht mehr als 10.

2. Verfahren zur Erzeugung eines behandelten Labdanumöls mit einem Säurewert von nicht mehr als 10, umfassend einen Schritt des Kontaktierens von zumindest einem Labdanum-Ausgangsmaterial, ausgewählt aus:
(a) Labdanumgummi, erhältlich durch Sammeln eines Harzes, getrennt von Cistus ladaniferus durch Sieden in Wasser,
(b) Labdanum-Extrakt, erhältlich durch Extrahieren des Labdanumgummis mit einem organischen Lösungsmittel, unter Entfernung von anorganischen Feststoffen, und
(c) Labdanumgummi-Destillat, erhältlich durch Destillation oder fraktionierte Destillation des Labdanumgummi-Extraktes,
mit einer alkalischen Substanz in einer Menge von 0,5-bis 10 Äquivalenten, bezogen auf das Säureäquivalent des Labdanum-Ausgangsmaterials.

3. Verfahren nach Anspruch 2, worin die alkalische Substanz ausgewählt ist aus Alkalimetallhydroxiden, Erdalkalimetallhydroxiden, Alkalimetallcarbonaten und einer Mischung von diesen.

4. Verfahren nach Anspruch 3, worin die alkalische Substanz Natriumhydroxid oder Kaliumhydroxid ist.

5. Verfahren nach einem der Ansprüche 2, 3 und 4, worin das Labdanum-Ausgangsmaterial mit der alkalischen Substanz durch Zugabe einer wäßrigen Lösung der alkalischen Substanz zu dem Labdanum-Ausgangsmaterial und anschließendes Mischen unter Rühren dieser Mischung kontaktiert wird.

6. Verfahren nach Anspruch 2, worin das Labdanum-Ausgangsmaterial mit der alkalischen Substanz in der Gegenwart eines organischen Lösungsmittels kontaktiert wird.

7. Verfahren nach Anspruch 6, worin das organischen Lösungsmittel ausgewählt ist aus Hexan, Heptan, Cyclohexan, Benzol, Toluol, Xylol und einer Mischung von diesen.

8. Parfümzusammensetzung, umfassend ein behandeltes Labdanumöl mit einem Säurewert von nicht mehr als 10.

## Revendications

1. Huile de labdanum traitée ayant un indice d'acide non supérieur à 10.

2. Procédé de préparation d'une huile de labdanum traitée ayant un indice d'acide non supérieur à 10 comprenant l'étape de mise en contact d'au moins une matière première de labdanum choisie parmi :
(a) la gomme de labdanum pouvant être obtenue en recueillant une résine séparée de Cistus ladaniferus en la faisant bouillir dans l'eau,
(b) l'extrait de labdanum pouvant être obtenu par extraction de ladite gomme de labdanum avec un solvant organique pour éliminer les solides minéraux et,
(c) le distillat de gomme de labdanum obtenu par distillation ou distillation fractionnée dudit extrait de gomme de labdanum,
avec une substance alcaline en une quantité de 0,5 à 10 équivalents, sur la base de l'équivalent d'acide de ladite matière première de labdanum.

3. Procédé selon la revendication 2, caractérisé en ce que ladite substance alcaline est choisie à partir des hydroxydes de métaux alcalins, des hydroxydes de métaux alcalino-terreux, des carbonates de métaux alcalins, et leur mélange.

4. Procédé selon la revendication 3, caractérisé en ce que ladite substance alcaline est l'hydroxyde de sodium ou l'hydroxyde de potassium.

5. Procédé selon l'une des revendications 2, 3 et 4, caractérisé en ce que ladite matière première de labdanum est mise en contact avec ladite substance alcaline en ajoutant une solution aqueuse de ladite substance alcaline à ladite matière première de labdanum, et ensuite en mélangeant sous agitation du mélange.

6. Procédé selon la revendication 2, caractérisé en ce que ladite matière première de labdanum est mise en contact avec ladite substance alcaline en présence d'un solvant organique.

7. Procédé selon la revendication 6, caractérisé en ce que ledit solvant organique est choisi parmi l'hexane, l'heptane, le cyclohexane, le benzène, le toluène, le xylène et un mélange de ceux-ci.

8. Composition de parfum contenant une huile de labdanum traitée ayant un indice d'acide non supérieur à 10.
